# EUROPÄISCHE PATENTSCHRIFT

(11) **EP 2 739 388 B1**
(45) Veröffentlichungstag und Bekanntmachung des Hinweises auf die Patenterteilung: **17.06.2015**
(21) Anmeldenummer: 12740994.4
(22) Anmeldetag: 01.08.2012
(51) Int. Cl.: B01J 19/24, C07C 5/48

(54) **REAKTOR ZUR DURCHFÜHRUNG EINER AUTOTHERMEN GASPHASENDEHYDRIERUNG**
REACTOR FOR CARRYING OUT AUTOTHERMAL GAS-PHASE DEHYDROGENATION
RÉACTEUR POUR LA RÉALISATION D'UNE DÉSHYDROGÉNATION AUTOTHERMIQUE EN PHASE GAZEUSE

(30) Priorität: 02.08.2011 EP 11176343
(43) Veröffentlichungstag der Anmeldung: 11.06.2014
(73) Patentinhaber: BASF SE, 67056 Ludwigshafen (DE)
(72) Erfinder: OLBERT, Gerhard, 69221 Dossenheim (DE); WEGERLE, Ulrike, 67550 Worms (DE); KOLIOS, Grigorios, 67435 Neustadt (DE); TELLAECHE HERRANZ, Carlos, 69126 Heidelberg (DE); HÖCHST, Reinhold, 67227 Frankenthal (DE); GIENGER, Andrea, 67149 Meckenheim (DE); BAUER, Roland, 67591 Offstein (DE)
(86) Internationale Anmeldenummer: PCT/EP2012/064988
(87) Internationale Veröffentlichungsnummer: WO 2013/017609

(56) Entgegenhaltungen:
- WO-A1-2011/067235
- WO-A1-2012/084609
- US-A1- 2008 119 673
- US-A1- 2009 292 030

## Beschreibung

Die Erfindung betrifft einen Reaktor zur Durchführung von autothermen Gasphasendehydrierungen unter Verwendung eines heterogenen Katalysators, der als Monolith ausgebildet ist sowie ein Verfahren unter Verwendung des Reaktors.

Keramische oder metallische Monolithe sind als Katalysatorträger für Edelmetallkatalysatoren in der mobilen und stationären Abgasreinigung etabliert. Die Kanäle bieten der Strömung einen geringen Strömungswiderstand bei gleichzeitig gleichmäßiger Zugänglichkeit der äußeren Katalysatoroberfläche für gasförmige Reaktionsmedien. Dies ist vorteilhaft gegenüber regellosen Haufwerken, bei denen durch unzählige Umlenkungen bei der Strömung um die Partikel ein großer Druckverlust entsteht und die Katalysatoroberfläche eventuell nicht gleichmäßig genutzt wird. Der Einsatz von Monolithen ist generell interessant für katalytische Prozesse mit hohen Volumenströmen und adiabater Reaktionsführung bei hohen Temperaturen. Diese Merkmale treffen in der chemischen Produktionstechnik insbesondere für Dehydrierungsreaktionen zu, die in einem Temperaturbereich von 400 °C bis zu 700 °C ablaufen.

Fortschritte in der Katalysatortechnik ermöglichen die selektive Verbrennung des Dehydrierwasserstoffes in Anwesenheit von Kohlenwasserstoffen, wie beispielsweise in US 7,034,195 beschrieben. Eine derartige Fahrweise wird als autotherme Dehydrierung bezeichnet und erlaubt, Dehydrierreaktoren direkt zu beheizen, so dass aufwändige Vorrichtungen zur indirekten Vor- und Zwischenheizung des Reaktionsgemisches entfallen. Ein derartiges Verfahren ist beispielsweise in US 2008/0119673 beschrieben. Dieses Verfahren besitzt jedoch den gravierenden Nachteil, dass die Dehydrierung an einem heterogenen Katalysator in Pelletform durchgeführt wird: Der hohe Strömungswiderstand von Pelletschüttungen erfordert einen großen Reaktorquerschnitt und eine entsprechend niedrige Durchströmungsgeschwindigkeit, um den Druckabfall in der katalytisch aktiven Schicht zu begrenzen. Dieser Nachteil wird durch eine sehr aufwändige Vorrichtung zur Dosierung und Verteilung des Sauerstoffes ausgeglichen, was den Vorteil der autothermen Dehydrierung beeinträchtigt.

Aus der nicht vorveröffentlichten europäischen Patentanmeldung EP 09 177 649.2 ist ein Reaktor sowie ein Verfahren zur autothermen Gasphasendehydrierung von Kohlenwasserstoffen unter Einsatz von als Monolithe ausgebildeten heterogenen Katalysatoren bekannt, das eine Beherrschung der brennbaren Reaktionsmedien bei den hohen Reaktionstemperaturen, häufig im Bereich von etwa 400 bis 700 °C, sowie eine einfache Zugänglichkeit und Handhabung der Monolithe, insbesondere beim Rüsten des Reaktors sowie bei einem Katalysatorwechsel, gewährleistet.

Die EP 09 177 649.2 stellt einen Reaktor in Form eines liegenden Zylinders zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches an einem heterogenen Katalysator, der als Monolith ausgebildet ist, zur Verfügung, wobei
- der Innenraum des Reaktors durch ein lösbar, in Längsrichtung des Reaktors angeordnetes kreiszylindrisches oder prismatisches, in Umfangsrichtung gasdichtes, an beiden Stirnseiten desselben offenes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist,
- mit einer oder mehreren Zuführleitungen für den zu dehydrierenden kohlenwasserstoffhaltigen Gasstrom in den Außenbereich B, Umlenkung des zu dehydrierenden Kohlenwasserstoffstroms an einem Ende des Reaktors und Zuführung über einen Strömungsgleichrichter in den Innenbereich A,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgemisch der autothermen Gasphasendehydrierung am Ende des Reaktors wie die Zuführleitung für den zu dehydrierenden Kohlenwasserstoffstrom.

An dem Reaktorende, an dem die Abführleitung für das Reaktionsgasgemisch der autothermen Gasphasendehydrierung angeordnet ist, ist vorteilhaft ein Rohrbündelwärmetauscher vorgesehen, mit einem Bündel von Rohren, durch die das Reaktionsgasgemisch zur autothermen Gasphasendehydrierung geleitet wird, sowie mit Zwischenräumen zwischen den Rohren, durch die, im Gegenstrom zum Reaktionsgemisch der autothermen Gasphasendehydrierung, der zu dehydrierende kohlenwasserstoffhaltige Gasstrom geleitet wird.

Ein aus sicherheitstechnischer Sicht verbesserter Reaktor ist in EP 10 196 216.5 beschrieben; und zwar ein Reaktor in Form eines liegenden Zylinders oder Prismas zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogegen Katalysator, der als Monolith ausgebildet ist, wobei
- der Innenraum des Reaktors durch ein lösbar, in Längsrichtung des Reaktors angeordnetes kreiszylindrisches oder prismatisches, gasdichtes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist, und wobei
   - an einem Reaktorende im Anschluss an das Gehäuse G ein Wärmetauscher vorgesehen ist
- mit einer oder mehreren Zuführleitungen für den zu dehydrierenden kohlenwasserstoffhaltigen Gasstrom,
   - mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
   - mit einer Abführleitung für das Reaktionsgasgemisch der autothermen Gasphasendehydrierung,
und wobei der Außenbereich B mit einem unter den Reaktionsbedingungen der autothermen Gasphasendehydrierung inerten Gas beaufschlagt ist und der zu dehydrierende kohlenwasserstoffhaltige Gasstrom über eine Zuführleitung in den Wärmetauscher eingeleitet, durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet.

Es handelt sich somit um einen Reaktor mit einem Reaktormantel, d.h. einer drucktragenden Hülle, die nicht mediumberührt ist, weder durch den kohlenwasserstoffhaltigen noch durch den sauerstoffhaltigen Strom.

Demgegenüber war es Aufgabe der vorliegenden Erfindung, den oben beschriebenen Reaktor, insbesondere bezüglich des Energieverbrauchs, weiter zu verbessern.

Die Aufgabe wird gelöst durch einen Reaktor in Form eines liegenden Zylinders oder Prismas zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogenen Katalysator, der als Monolith ausgebildet ist, wobei
- der Innenraum des Reaktors durch ein in Längsrichtung des Reaktors angeordnetes kreiszylindrisches oder prismatisches gasdichtes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher vorgesehen ist
- mit einer oder mehreren Zuführleitungen für den zu dehydrierenden kohlenwasserstoffhaltigen Gasstrom,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagbar ist und
- der zu dehydrierende kohlenwasserstoffhaltige Gasstrom über eine Zuführleitung in den Wärmetauscher eingeleitet, im Wärmetauscher durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet,
der dadurch gekennzeichnet ist, dass der Innenbereich A gegenüber dem Außenbereich B des Reaktors mittels eines mikroporösen Hochleistungsdämmstoffes mit einem Wärmeleitfähigkeitswert λ bei Temperaturen bis 700 °C von kleiner als 0,05 W/m*K isoliert ist.

Es wurde gefunden, dass sich die Wärmeverluste des in EP 10 196 216.5 beschriebenen Reaktors entscheidend verringern lassen, indem der Innenbereich A des Reaktors, in den Eduktströme, d.h. der zu dehydrierende kohlenwasserstoffhaltige Gasstrom und der Sauerstoff enthaltende Gasstrom, zugeführt, miteinander vermischt werden und unter Erhalt eines Reaktionsgasgemisches reagieren, das in indirektem Wärmetausch den kohlenwasserstoffhaltigen Edukt-Gasstrom vorheizt und anschließend aus dem Reaktor austritt, gegen den Außenbereich B des Reaktors unter Einsatz eines mikroporösen Hochleistungsdämmstoffes isoliert wird. Da die autotherme Gasphasendehydrierung im Innenbereich A des Reaktors bei hohen Temperaturen von etwa 400 bis 700 °C stattfindet, ist die thermische Isolation desselben gegenüber dem Außenbereich B von überragender Bedeutung.

Erfindungsgemäß werden hierfür mikroporöse Hochleistungswerkstoffe eingesetzt, mit Wärmeleitfähigkeitswerten λ bei Temperaturen bis 700 °C von < 0,05 W/m*K. Derartige Werkstoffe sind im VDI-Wärmeatlas, 9. Auflage 2002, Abschnitt Kf 8, als kontinuierlich aufgebaute Superisolationen beschrieben. Superisolationen sind demnach Wärmeisolationen, deren Gesamtwärmedurchlässigkeit deutlich kleiner ist als diejenige der ruhenden Luft.

Die erfindungsgemäße Isolation aus einem mikroporösen Hochleistungsdämmstoff hat bei gleichem Wärmedurchgangskoeffizienten gegenüber konventionellen Faserisolationen eine wesentlich geringere Wärmekapazität sowie ein wesentlich kleineres Volumen. Eine niedrige Wärmekapazität ist jedoch unabdingbar für die Temperaturkontrolle während des Abbrennvorgangs in der Regenerierung des Katalysators, insbesondere das niedrige Volumen begünstigt darüber hinaus die Integration aller temperaturbelasteten Bauteile in ein einziges gemeinsames Gehäuse.

Als mikroporöse Hochleistungsdämmstoffe können insbesondere Werkstoffe aus silikatischen Substanzen eingesetzt werden. Insbesondere handelt es sich dabei um hochdisperse Kieselsäure als Hauptbestandteil und einem Trübungsmittel zur Minimierung der Infrarotstrahlung als weiteren Bestandteil. Als Trübungsmittel kann auch ein Gemisch eingesetzt werden. Die mikroporösen Hochleistungsdämmstoffe liegen insbesondere in Form von mikroporösen Partikeln mit einer durchschnittlichen Porengröße von etwa 20 nm vor.

Derartige Werkstoffe sind beispielsweise als WDS^{®} Ultra der Firma Porextherm bekannt: WDS^{®} Ultra enthält als Hauptbestandteil ca. 80 % Siliziumdioxid und daneben ca. 15 % Siliziumcarbid und gewährleistet einen besonderes niedrigen Wärmeübergang sowohl durch Wärmeleitung, durch Konvektion als auch durch Wärmestrahlung:

Aufgrund der Zellstruktur des eingesetzten mikroporösen Materials und der Tatsache, dass es sich um kugelförmige Materialpartikel handelt, sind die Kontaktpunkte zwischen den Teilchen unendlich klein, mit dem Ergebnis einer sehr niedrigen Festkörperleitfähigkeit.

Der Wärmeübergang durch Gaswärmeleitung ist ebenfalls sehr niedrig. Aufgrund der Zellstruktur des mikroporösen Materials, mit einer durchschnittlichen Porengröße von 20 nm, die kleiner ist als die mittlere freie Weglänge der Gasmoleküle, führt dies hauptsächlich zu Kollisionen der Gasmoleküle mit den Porenwänden, wodurch der Energieaustausch zwischen den einzelnen Molekülen auf ein Minimum reduziert wird.

Der Wärmeübergang durch Wärmestrahlung findet durch elektromagnetische Wellen statt und gewinnt bei steigender Temperatur, insbesondere oberhalb von 400 °C an Bedeutung. Durch das Hinzufügen von infrarotabsorbierenden Stoffen (Trübungsmittel) zu der mikroporösen Materialmischung wird auch diese Art der Wärmeübertragung erheblich eingeschränkt.

Aus den obigen Gründen kann mit den erfindungsgemäß eingesetzten mikroporösen Dämmstoffen eine sehr viel höhere Isolierwirkung gegenüber herkömmlichen Isolierwerkstoffen, wie Mineralfasern, Feuerleichtsteinen oder anorganischen Dämmplatten erreicht werden. So können bei gleicher Isolierwirkung zum Beispiel die Schichtdicke um den Faktor 6 und das Gewicht um den Faktor 2 bis 15 reduziert werden.

Eine weitere Verbesserung kann durch Einsatz der Vakuumtechnologie erreicht werden, indem evakuierte Platten oder aus Platten gebildete Formteile eingesetzt werden.

Insbesondere sind das Gehäuse G, der Wärmetauscher, die Zuführleitung für den zu dehydrierenden kohlenwasserstoffhaltigen Gasstrom, sowie die Zuführleitungen für den Sauerstoff enthaltenden Gasstrom gegenüber dem Außenbereich B des Reaktors mittels des mikroporösen Hochleistungsdämmstoffes isoliert.

Der mikroporöse Hochleistungsdämmstoff wird vorteilhaft in Form von Platten oder aus Platten hergestellten Formteilen eingesetzt.

Bevorzugt sind die Platten oder aus Platten hergestellten Formteile mit einer Schicht aus einem Werkstoff ummantelt, die die mechanische Stabilität derselben erhöht.

Vorteilhaft kann hierzu als Werkstoff Metall, besonders bevorzugt Edelstahl oder Aluminium eingesetzt werden.

Die aus Platten aus dem mikroporösen Hochleistungsdämmstoff hergestellten Formteile können vorteilhaft miteinander verzahnbar ausgebildet sein, dergestalt, dass sie unter mechanischer und thermischer Belastung stets eine durchgehende Isolation gewährleisten.

In Längsrichtung des Reaktors ist ein zylindrisches oder prismatisches Gehäuse G angeordnet, das den Innenraum des Reaktors in einen Innenbereich A und einen konzentrisch um denselben angeordneten Außenbereich B aufteilt.

Der Außenbereich B ist mit einem unter den Reaktionsbedingungen der autothermen Gasphasendehydrierung inerten Gas beaufschlagt, d.h. einem Gas oder Gasgemisch, das an der Reaktion der autothermen Gasphasendehydrierung nicht unmittelbar beteiligt ist, insbesondere ausgewählt aus Wasser, Kohlendioxid, Stickstoff und Edelgasen oder Gemischen hiervon. Bevorzugt wird als unter den Reaktionsbedingungen der autothermen Gasphasendehydrierung inertes Gas Wasserdampf eingesetzt, da dieser in einfacher Weise, durch Auskondensieren, aus dem Reaktionsgasgemisch wieder abgetrennt werden kann.

Bevorzugt wird das unter den Reaktionsbedingungen der autothermen Gasphasendehydrierung inerte Gas als Purge-Gasstrom mit einem geringen Massenstrom im Vergleich zum Massenstrom des kohlenwasserstoffhaltigen Gasstromes, d.h. einem Massenstrom von 1/5 bis 1/100, bevorzugt einem Massenstrom von 1/10 bis 1/50, bezogen auf den Massenstrom des kohlenwasserstoffhaltigen Gasstromes, unter geringem Überdruck von 2 bis 50 mbar, bevorzugt von 25 bis 30 mbar, bezogen auf den Druck im Innenbereich A durch denselben geleitet.

Der Purge-Gasstrom kann vorteilhaft durch den Außenbereich B geleitet werden, indem er an einem Reaktorende über eine oder mehrere Zuführleitungen in den Außenbereich B des Reaktors eingeleitet und am entgegengesetzten Reaktorende in den Innenbereich A des Reaktors weitergeleitet wird, bevorzugt über eine oder mehrere, vorteilhaft in einem Winkel verschieden von 90° zur Zuführleitung, für den zu dehydrierenden kohlenwasserstoffhaltigen Gasstrom angeordnete Verbindungsleitung(en).

Die eine oder mehrere Verbindungsleitung(en), die den Purge-Gasstrom aus dem Außenbereich B in den Innenbereich A leiten, sind bevorzugt rückströmungsfrei ausgebildet, beispielsweise durch eine gewendelte Form derselben. Der Einlass aus dem Außenbereich B in die Verbindungsleitung für den Purge-Gasstrom, soll bevorzugt so hoch wie möglich im Außenbereich B des Reaktors angeordnet sein.

Der Purge-Gasstrom spült permanent den Außenbereich B des Reaktors und hält diesen frei von Komponenten des Reaktionsgasgemischs.

An einem Ende des Gehäuses G ist ein Wärmetauscher angeschlossen, der insbesondere ein Rohrbündelwärmetauscher oder ein Plattenwärmetauscher sein kann. Für den Fall des Rohrbündelwärmetauschers ist der Anschluss zwischen demselben und dem Gehäuse G so ausgebildet, dass der Innenbereich A mit dem Innenraum der Rohre des Rohrbündelwärmetauschers kommuniziert. Für den Fall eines Plattenwärmetauschers kommuniziert der Innenbereich A des Reaktors mit den Spalten zwischen den Platten des Plattenwärmetauschers.

Der Zwischenraum zwischen den Rohren des Rohrbündelwärmetauschers bzw. zwischen jeweils zwei zu einer Wärmetauscherplatte zusammengeschweißten Blechen des Plattenwärmetauschers ist über eine Leitung, die an das dem Wärmetauscher entgegengesetzte Ende des Reaktors führt und dort umgelenkt wird, mit dem dem Wärmetauscher entgegengesetzten Ende des Gehäuses G und somit dem Innenbereich des Reaktors gasdicht gegenüber dem Außenbereich B verbunden.

Der kohlenwasserstoffhaltige Strom wird durch den Zwischenraum zwischen den Rohren des Rohrbündelwärmetauschers bzw., für den Fall eines Plattenwärmetauschers, durch die Zwischenräume zwischen den jeweils eine Wärmetauscherplatte bildenden Blechen geleitet, mit dem im Gegenstrom durch die Rohre bzw. durch die Spalte zwischen den Platten des Plattenwärmetauschers zirkulierenden Produktgasstrom aufgeheizt, an das entgegengesetzte Ende des Reaktors geführt, dort umgelenkt und in den Innenbereich A des Gehäuses eingeleitet.

Die autotherme Gasphasendehydrierung findet an einem heterogenen Katalysator statt, der in Form von Monolithen vorliegt.

Als Monolith wird vorliegend ein einstückiger, parallelepipedischer Block mit einer Vielzahl von parallel zueinander angeordneten, durchgehenden Kanälen mit engem Querschnitt, im Bereich von etwa 0,5 bis 4 mm, verstanden.

Die Monolithe sind bevorzugt aus einem keramischen Werkstoff als Trägermaterial gebildet, worauf eine katalytisch aktive Schicht, bevorzugt nach dem sogenannten Wash-Coating-Verfahren, aufgebracht ist.

Die nebeneinander, übereinander und hintereinander zu einer Packung gestapelten Monolithe sind bevorzugt in einer Blähmatte oder in einem Mineralfaservlies eingehüllt und in eine Einhausung mit Verspanneinrichtung eingesetzt. Als Mineralfaservliese werden bevorzugt Vliese eingesetzt, wie sie für den Einsatz für Abgaskatalysatoren bekannt sind, beispielsweise Interam^{®} Lagermatten der Firma 3M^{®}.

Blähmatten sind aus der katalytischen Abgasreinigung bekannt und beispielsweise in DE-A 40 26 566 beschrieben: Sie bestehen im Wesentlichen aus Keramikfasern mit Glimmereinlagerung. Infolge der Glimmereinlagerung hat die Blähmatte bei steigenden Temperaturen das Bestreben sich auszudehnen, wodurch eine besonders sichere Halterung des darin eingehüllten Körpers auch bei höheren Temperaturen erreicht wird.

Die Mineralvliese oder Blähmatten werden so ausgewählt, dass sie sich unter Wärmeeinwirkung ausdehnen und dass sie die in der Regel keramischen Monolithe gegen das Gehäuse abdichten, insbesondere eine Reibung der Monolithe am Gehäuse sowie eine Bypassströmung des Reaktionsgasgemisches an der Innenwand des Gehäuses verhindern.

Die Blähmatten, in die die Monolithe eingehüllt sind, sorgen für eine stabile Position derselben, da sie unter Wärmeausdehnung eine Spannkraft erzeugen. Im Zuge von Fehlbedingungen kann die Spannkraft jedoch nachlassen. Daher kann vorteilhaft eine Verspanneinrichtung vorgesehen werden: Hierzu werden die Blähmatten an ihrem dem Austritt des Reaktionsgasgemisches entsprechenden Ende mit einem aus einem hochtemperaturfesten Gewebe, das beispielsweise metallisch sein kann, gebildeten U-Profil eingefasst. In Verlängerung der Blähmatten werden Metallprofile angeordnet, die mit einem Querschnitt entsprechend dem Querschnitt der Blähmatten an denselben ansetzen und sich in Strömungsrichtung des Reaktionsgasgemisches zunehmend verbreitern. Dadurch wirken die Metallprofile als Stütze gegen das Verschieben der Blähmatten in Strömungsrichtung des Reaktionsgasgemisches.

Die in Blähmatten eingehüllten Monolithe sind in einem Gehäuse angeordnet.

Das Gehäuse ist vorteilhaft aus einem Werkstoff gebildet, der bei der hohen Belastung durch die Reaktionstemperatur, häufig im Bereich von etwa 400 bis 700 °C, mechanisch und chemisch stabil ist und auch keine katalytische Aktivität für die autotherme Gasphasendehydrierung aufweist.

Bevorzugt ist das Gehäuse aus einem Werkstoff gebildet, der hitzebeständig ist, insbesondere aus einem Edelstahl mit der Werkstoffnummer 1.4541, 1.4910 oder 1.4841.

Das Gehäuse soll möglichst dünn sein, um eine möglichst geringe Wärmekapazität zu haben, und somit die Wärmeverluste zwischen dem Außenbereich B und dem Innenbereich A zu begrenzen.

Das Gehäuse kann bevorzugt thermisch isoliert sein.

Das Gehäuse kann bevorzugt lose im Reaktor gelagert sein.

Das Gehäuse ist bevorzugt als Quader ausgebildet.

Bevorzugt sind die Seitenwände des als Quader ausgebildeten Gehäuses einzeln abnehmbar ausgebildet, dergestalt, dass eine komplette Packung oder einzelne Monolithe einer Packung aus einer katalytisch aktiven Zone ausgetauscht werden können.

Die einzelnen Monolithe werden nebeneinander, übereinander und hintereinander, in der erforderlichen Anzahl gestapelt, um eine katalytisch aktive Zone auszufüllen, unter Ausbildung einer Packung.

Vor jeder Packung ist jeweils eine Mischzone mit festen Einbauten, die nicht katalytisch aktiv sind, vorgesehen. In der Mischzone erfolgt die Vermischung des kohlenwasserstoffhaltigen Gasstromes mit dem Sauerstoff enthaltenden Strom, wobei in der in Strömungsrichtung zuerst angeströmten Mischzone die Vermischung des Sauerstoff enthaltenden Gasstromes mit dem kohlenwasserstoffhaltigen Einsatzstrom erfolgt und in den darauffolgend angeströmten Mischzonen jeweils eine Zwischenspeisung eines Sauerstoff enthaltenden Gasstromes in das noch zu dehydrierende, Kohlenwasserstoff enthaltende Reaktionsgemisch erfolgt.

Der zu dehydrierende kohlenwasserstoffhaltige Gasstrom kann bevorzugt an zwei oder mehreren Stellen in den Wärmetauscher eingeleitet werden, insbesondere als ein Hauptstrom mit höherem Massenstrom und ein oder mehrere Nebenströme mit niedrigerem Massenstrom gegenüber dem Hauptstrom.

Für die Aufheizung des zu dehydrierenden kohlenwasserstoffhaltigen Gasstromes können zusätzlich zum Wärmetauscher eine oder mehrere Zusatzheizungen vorgesehen sein. Als Zusatzheizung kann bevorzugt die Zuführung von Wasserstoff durch die Zuführleitung für den zu dehydrierenden kohlenwasserstoffhaltigen Gasstrom möglichst nahe am Eintritt in die Mischzonen, die vor jeder katalytisch aktiven Zone angeordnet sind, vorgesehen sein.

Alternativ oder zusätzlich kann als Zusatzheizung eine elektrische Heizung vorgesehen sein, die bevorzugt lösbar, als Einstecksystem, innerhalb des Außenbereichs B des Reaktors in die Zuführleitung für den kohlenwasserstoffhaltigen Gasstrom, nach dem Austritt desselben aus dem Wärmetauscher eingebracht wird. Alternativ oder zusätzlich kann als Zusatzheizung ein Muffelbrenner vorgesehen sein.

Durch die bevorzugte Ausbildung des Reaktors als liegender Zylinder ist der Innenraum A, der die Monolith-Packungen umfasst, großflächig abgestützt und dadurch mechanisch entlastet. Darüber hinaus ist bei dieser Reaktoranordnung die Zugänglichkeit zu den einzelnen Monolithpackungen einfacher.

Der Mantel des Reaktors ist bevorzugt aus einem für Druckbehälter zugelassenen legierten Stahl gebildet, insbesondere aus Schwarzstahl, bevorzugt aus Kesselblech HII, oder aus einem legierten Stahl mit der Werkstoffnummer 1.4541 oder 1.4910. Der Mantel des Reaktors kann auch mit Schamotteausmauerung ausgekleidet sein.

Jede Mischzone umfasst bevorzugt jeweils einen Rohrverteiler, gebildet aus einer Vielzahl von parallel zueinander, in einer Ebene senkrecht zur Längsrichtung des Reaktors angeordneten Einsteckrohren, die mit einer oder mehreren der Verteilerkammern verbunden sind, und die eine Vielzahl von gleichmäßig zueinander beabstandeten Austrittsöffnungen für den Sauerstoff enthaltenden Gasstrom aus dem Einsteckrohr aufweisen, sowie eine Vielzahl von gleichmäßig zueinander beabstandeten Mischkörpern.

Die Mischkörper können vorteilhaft als Mischplatten ausgebildet sein.

Der Wärmetauscher ist bevorzugt ein Rohrbündelwärmetauscher.

Der Rohrbündelwärmetauscher ist vorteilhaft aus einem hoch warmfesten Edelstahl, insbesondere einem Edelstahl mit der Werkstoffnummer 1.4541 oder 1.4910, gebildet. Die Rohre des Rohrbündelwärmetauschers sind an beiden Enden derselben in Rohrböden vorteilhaft spaltfrei durch Hinterbodenschweißung eingebracht und die Rohrböden des Rohrbündelwärmetauschers auf der Heißgasseite derselben mit einem hitzebeständigen Edelstahl, insbesondere mit einem Edelstahl mit der Werkstoffnummer 1.4841, plattiert.

Bevorzugt ist an der Stirnseite des Gehäuses G, an der der kohlenwasserstoffhaltige Gasstrom in den Innenbereich A eingeleitet wird, ein Strömungsgleichrichter angeordnet.

Gegenstand der Erfindung ist auch ein Verfahren zur Durchführung von autothermen Dehydrierungen unter Verwendung des vorstehend beschriebenen Reaktors.

In einer bevorzugten, voll kontinuierlichen Fahrweise können zwei oder mehrere Reaktoren eingesetzt werden, wobei mindestens ein Reaktor für die autotherme Gasphasendehydrierung genutzt und gleichzeitig mindestens ein weiterer Reaktor regeneriert wird.

Die autotherme Gasphasendehydrierung ist bevorzugt eine autotherme Dehydrierung von Propan, Butan, i-Butan oder Buten.

Der erfindungsgemäße Reaktor und das erfindungsgemäße Verfahren weisen insbesondere den Vorteil auf, dass die Energiebilanz der autothermen Gasphasendehydrierung durch Einsatz eines Superisolators aus einem mikroporösen Hochleistungsdämpfstoff gegenüber herkömmlichen Reaktoren entscheidend verbessert ist.

Die Erfindung wird im Folgenden anhand eines Ausführungsbeispiels sowie einer Zeichnung näher erläutert.

Es zeigen im Einzelnen:
- Figur 1: einen Längsschnitt in vertikaler Ebene durch eine bevorzugte Ausführungsform eines erfindungsgemäßen Reaktors in Form eines liegenden Zylinders,
- Figur 2: einen Längsschnitt durch eine bevorzugte Ausführungsform eines erfindungsgemäßen Reaktors in Form eines aufrechtstehenden Zylinders, mit vertikaler Längsachse,
- Figur 3: einen Längsschnitt durch den in Figur 1 dargestellten Reaktor in horizontaler Ebene,
- Figur 4: einen Querschnitt durch den in Figur 1 dargestellten Reaktor in der Ebene C-C,
- Figur 5: einen Querschnitt durch den in Figur 1 dargestellten Reaktor in der Ebene D-D,
- Figur 6: einen Querschnitt durch den in Figur 1 dargestellten Reaktor in der Ebene E-E,
- Figur 7: einen Querschnitt durch den in Figur 1 dargestellten Reaktor in der Ebene F-F, und
- Figur 8: eine schematische Darstellung der Wärmeleitfähigkeitswerte in Abhängigkeit von der Temperatur für mikroporöse Hochleistungsdämmstoffe WDS^{®} der Fa. Porextherm im Vergleich zu herkömmlichen Dämmstoffen.

In den Figuren bezeichnen gleiche Bezugszeichen jeweils gleiche oder entsprechende Merkmale.

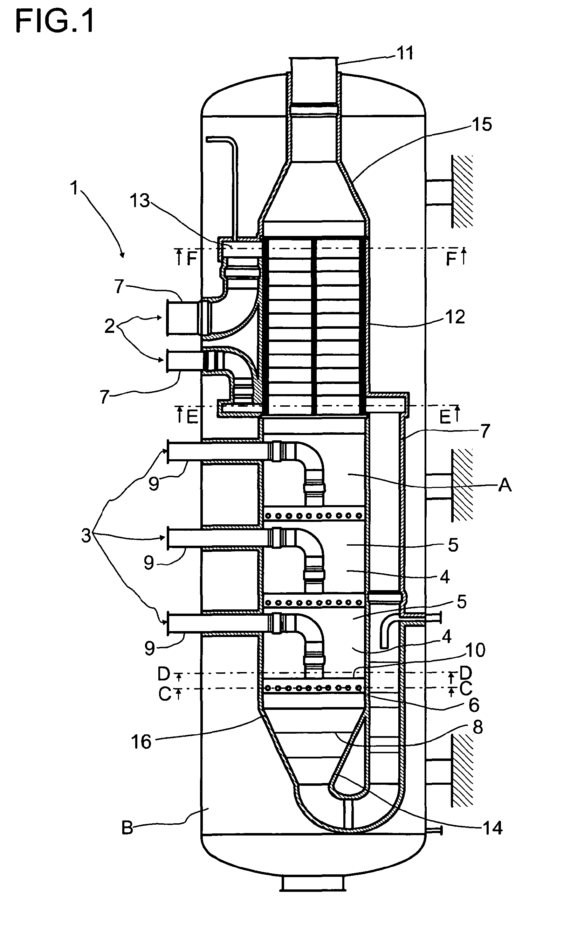

Der Längsschnitt in vertikaler Ebene in Figur 1 zeigt schematisch eine bevorzugte Ausführungsform eines erfindungsgemäßen Reaktors 1, der mit einem zu dehydrierenden kohlenwasserstoffhaltigen Gasstrom 2 über eine Zuführleitung 7 und über Zuführleitungen 9 mit Verteilerkammern 10 mit einem Sauerstoff enthaltenden Gasstrom 3 gespeist wird.

Figur 1 zeigt, dass das Gehäuse G den Innenraum des Reaktors 1 in einen Innenbereich A und einen Außenbereich B aufteilt. Im Innenbereich A sind beispielhaft drei katalytisch aktive Zonen 5 angeordnet, worin jeweils eine Packung aus aufeinander, nebeneinander und hintereinander gestapelten, nicht im Detail dargestellten Monolithen 4 und vor jeder katalytisch aktiven Zone 5 jeweils eine Mischzone 6 mit festen Einbauten vorgesehen ist. Der zu dehydrierende kohlenwasserstoffhaltige Gasstrom 2 wird über einen Wärmetauscher 12 geleitet, durch indirekten Wärmetausch mit dem Reaktionsgasgemisch aufgeheizt und weiter an das entgegengesetzte Ende des Reaktors geleitet, dort umgelenkt, über einen Strömungsgleichrichter 8 in den Innenbereich A eingeleitet, in den Mischzonen 6 mit dem Sauerstoff enthaltenden Gasstrom 3 vermischt, worauf in den katalytisch aktiven Zonen 5, die mit Monolithen 4 bestückt sind, die autotherme Gasphasendehydrierung stattfindet. Das Reaktionsgasgemisch wird über einen Sammelkasten 13 am Ausgang des Wärmetauschers 12 über die Abführleitung 11 aus dem Reaktor abgezogen.

Die Bezugsziffer 14 bezeichnet den Übergang von einer konischen auf eine quaderförmige Geometrie beim Eingang zum Innenbereich A und die Bezugsziffer 15 den Übergang von quaderförmig auf konisch beim Produktgasaustritt aus dem Wärmetauscher 12.

Mit der Bezugsziffer 16 ist der mikroporöse Hochleistungsdämmstoff bezeichnet, der schraffiert dargestellt ist, und der den Innenbereich A im Reaktor 1 gegenüber dem Außenbereich B durchgehend isoliert.

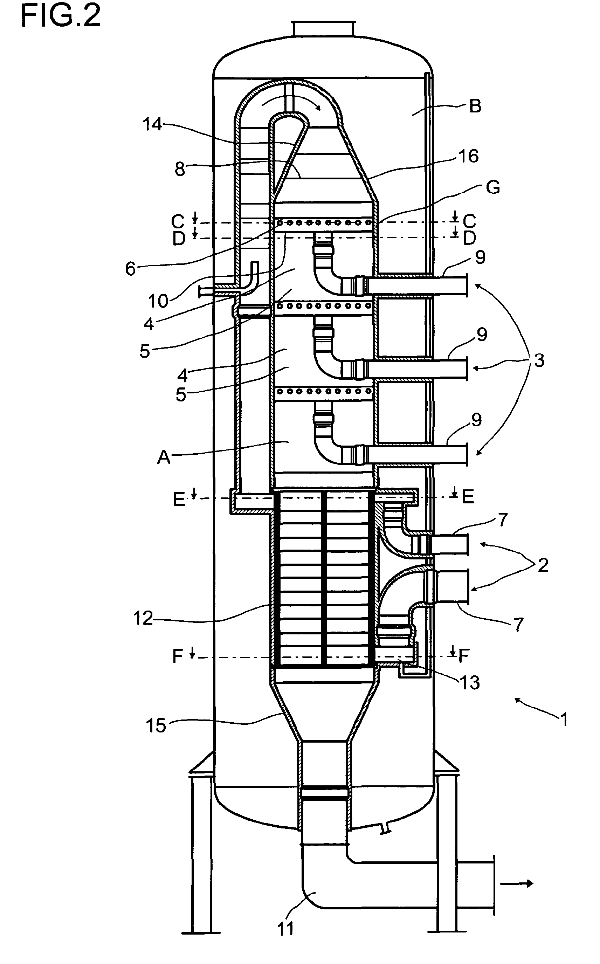

Figur 2 zeigt einen wie in Figur 1 dargestellten analogen Reaktor, der jedoch aufrecht stehend, mit vertikaler Längsachse, ausgebildet ist.

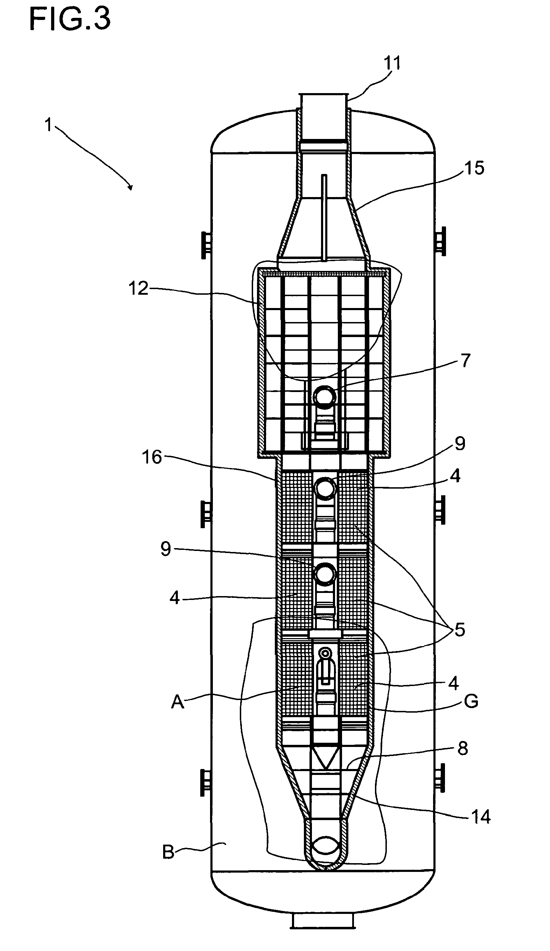

Figur 3 zeigt denselben wie in Figur 1 dargestellten Reaktor, jedoch als Längsschnitt in horizontaler Ebene.

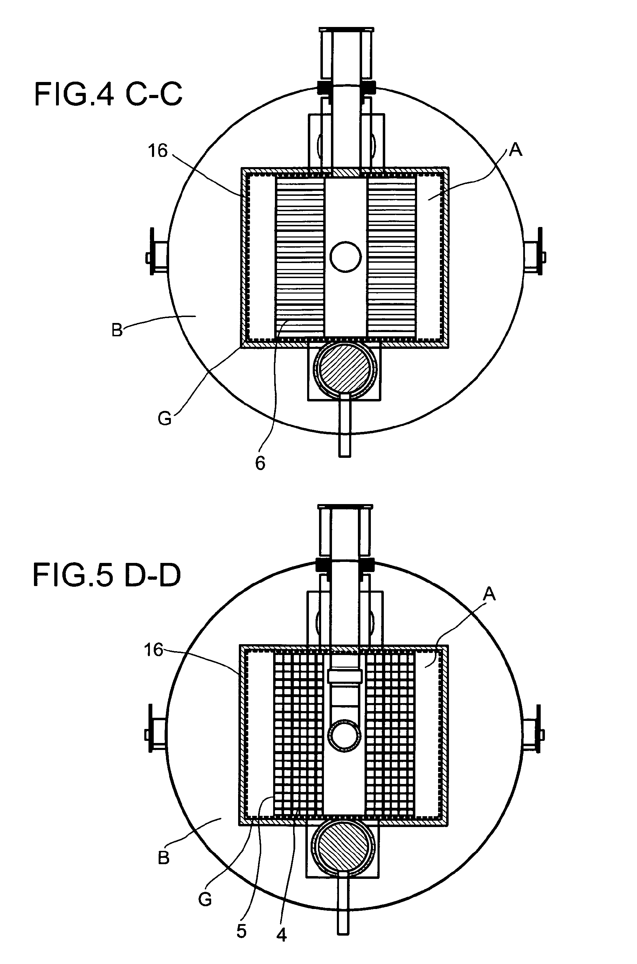

Figur 4 zeigt einen Querschnitt durch den in den Figuren 1 und 2 dargestellten Reaktor im Bereich der Mischzone 6 (Schnitt C-C),

Figur 5 einen Querschnitt durch denselben Reaktor im Bereich der katalytisch aktiven Zone 5 (Schnitt D-D),

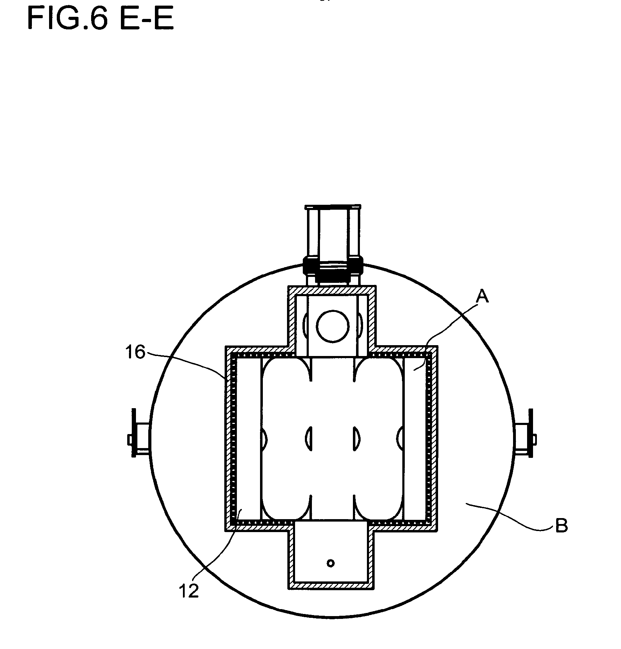

Figur 6 einen Querschnitt durch denselben Reaktor im Bereich des Reaktionsgaseintritts in den Wärmetauscher 12,

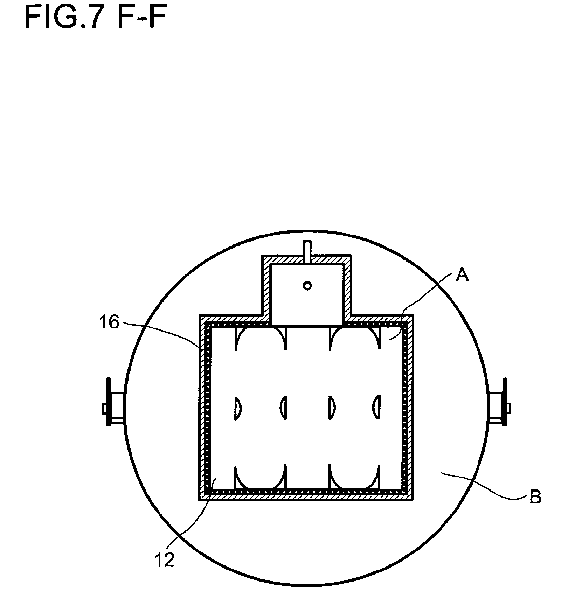

Figur 7 einen Querschnitt durch denselben Reaktor im Bereich des Reaktionsgasaustrittes aus dem Wärmetauscher 12.

In sämtlichen Querschnittsdarstellungen (Figuren 4 bis 7) ist die Isolation aus dem mikroporösen Hochleistungsdämmstoff (Bezugsziffer 16) zu erkennen, die den Innenbereich A gegenüber dem Außenbereich B isoliert.

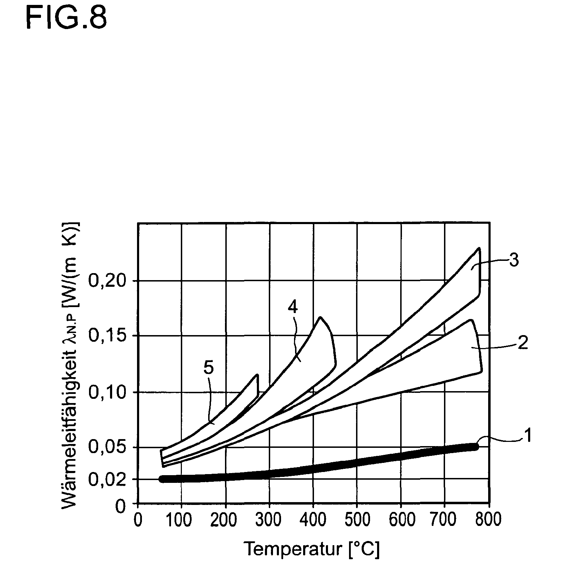

Figur 8 verdeutlicht die extrem niedrigen Wärmeleitfähigkeitswerte für die erfindungsgemäß eingesetzten mikroporösen Hochleistungsdämmstoffe, z.B. WDS^{®} der Fa. Porextherm, Kurve 1, im Vergleich zu gängigen Dämmstoffen, d.h. Kurve 2 für Silizium-Silikat-Platten, Kurve 3 für Calcium-Magnesium-Silikatfaser-Matten, Kurve 4 für Glaswolle (Platten und Matten) und für Steinwolle (Matten), wobei die Wärmeleitfähigkeiten A in W/m*K auf der Ordinate gegen die Temperatur in °C auf der Abszisse aufgetragen sind.

Die Erfindung wird im Folgenden anhand eines Ausführungsbeispiels näher erläutert. Es wird beispielhaft im Detail eine bevorzugte Ausführungsform für einen Reaktor 1 in großtechnischem Maßstab beschrieben:

### Ausführungsbeispiel

Die Abmessungen der Außenhülle (liegender Zylinder mit Klöpperbodenenden) betragen:

| | |
|---|---|
| Länge: | 24500 mm, |
| Durchmesser: | 6000 mm, |

### Werkstoff der Außenhülle: HII

### Auslegungsdaten der Außenhülle:

| | |
|---|---|
| Druck: | 7 bara |
| Temperatur: | 350 °C. |

Der Reaktor besteht aus folgenden Einzelkomponenten (Werkstoff 1.4541):
- 1 Stück Zuleitung Eduktgas DN1000 (Bezugsziffer 7 in Fig. 1) (Hauptstrom 80-100 % regelbar) durch Reaktoraußenwand zum integrierten Wärmetauscher (Bezugsziffer 12 in Fig. 1).
- 1 Stück Wärmetauscher in Rohrbündelausfertigung integriert, Bezugsziffer 12 Eduktgas strömt mantelseitig im Kreuzgegenstrom; Produktgas strömt durch 11000 Rohre 20 mm x 2 mm x 6000 mm; Dreiecksteilung 26 mm; Rohre sind verteilt auf zwei rechteckige Bereiche mit den jeweiligen Abmessungen von 3370 mm x 1000 mm. Die Einschweißung der Rohre an den Rohrboden ist als Hinterbodenausführung ausgeführt. Der Wärmetauscher hat auf der kalten Seite einen Kompensator in Schwimmkopfausführung.
- 1 Stück Zuleitung Eduktgas DN600 (Bypassstrom 0 - 20 % regelbar) durch die Reaktoraußenwand zum integrierten Wärmetauscher.
- 1 Stück Verbindungsleitung Eduktgas DN1000 vom Austritt aus integriertem Wärmetauscher 12 zum Reaktoreingang.
   In der Verbindungsleitung ist eine elektrische Zusatzheizung eingebaut.
- 1 Stück Zuleitung DN200 und Einmischung von Heizgas 17 in die Verbindungsleitung zwischen Wärmetauscher 12 und Eingang in den Innenbereich A; Einmischung als perforiertes Rohrsystem ausgebildet. Als Heizgas wird Wasserstoff verwendet.
- 1 Stück Übergang 14 konisch von DN1000 rund auf quaderförmig (Eintritt in das Gehäuse G) 3370 mm x 2850 mm mit 3 integrierten Lochblechen als Strömungsgleichrichter 8.
- 3 Stück Mischzonen 6 mit festen Einbauten vor jeder katalytisch aktiven Zone 5 bestehend aus jeweils 20 Verteilerrohren DN125.
- 1 Stück Gehäuse G zur Lagerung der Monolithe 4
   Abmessungen: 3370 mm x 2850 mm x 8500 mm
   Die Monolithe sind zu größeren Packungen zwecks einfacherem Einbau vormontiert. Eine Packung besteht aus 6 x 4 Monolithen, die durch Blähmatten beabstandet sind. Die Matten sind stirnseitig in Strömungsrichtung mit einem verklebten Metallsieb gegen Zerfall und Abrasion geschützt. Die einzelnen Monolithe 4 sind mit Abstandhalter gegen Verschiebung gesichert.
   Jedes Packungsmodul wird außen über einen stabilen Kasten unter Spannung gehalten. Die einzelnen Packungsmodule sind als Einschubmodule ausgebildet. Die Abdichtung der Einschubmodule zum eigentlichen Reaktorgehäuse erfolgt über eine dünne Dichtmatte.
- 3 Stück katalytisch aktive Zonen 5, die entsprechend mit den Packungsmodulen aus Monolithen 4 bestückt sind.
   Jede katalytisch aktive Zone 5 besteht aus 15 hintereinander gesetzten Monolithreihen. Jede Monolithreihe besteht aus 240 Monolithen. Insgesamt besteht eine katalytisch aktive Zone 5 aus 3600 Monolithen mit den Abmessungen 150 mm x 150 mm x 150 mm.
   Die einzelnen Monolithreihen sind beabstandet. In diese Beabstandung sind entsprechende Temperaturmessungen zur Regelung und Steuerung des Prozesses eingebaut.
- 1 Stück Übergang Gehäuse G auf integrierten Wärmetauscher 12 Abmessungen : 3370 mm x 2850 mm x 700 mm; rechteckig Der Übergang ist in einer Schweißkonstruktion ausgeführt.
- 1 Stück Übergang 15 für das Reaktionsgasgemisch aus integriertem Wärmetauscher 12 von rechteckig 3370 mm x 2850 mm auf rund DN1500; Ausführung des Übergangs konisch.
- 1 Stück Austrittsleitung 11 DN1500 für das Reaktionsgasgemisch durch die Reaktoraußenwand.
- 3 Stück Zuleitungen DN600 9 für O₂-haltiges Gas durch die Reaktoraußenwand zur Verteilerkammer 10 der jeweiligen katalytisch aktiven Zone 5.

Alle Inneneinbauten sind mit einem mikroporösen Hochleistungsdämmstoff (Superisolation) 16, z.B. aus WDS^{®} der Fa. Porextherm, für den Innenbereich A gegenüber dem Außenbereich B versehen. Diese Superisolation ist gegen Abrieb und Feuchtigkeit mit einer metallischen Ummantelung (Kapselung) geschützt. Der drucktragende Reaktormantel ist außen isoliert.

## Patentansprüche

1. Reaktor (1) in Form eines Zylinders oder Prismas zur Durchführung einer autothermen Gasphasendehydrierung eines kohlenwasserstoffhaltigen Gasstromes (2) mit einem Sauerstoff enthaltenden Gasstrom (3) unter Erhalt eines Reaktionsgasgemisches, an einem heterogenen Katalysator, der als Monolith (4) ausgebildet ist, wobei
- der Innenraum des Reaktors (1) durch ein in Längsrichtung des Reaktors (1) angeordnetes kreiszylindrisches oder prismatisches gasdichtes Gehäuse G in
- einen Innenbereich A, mit einer oder mehreren katalytisch aktiven Zonen (5), worin jeweils eine Packung aus aufeinander, nebeneinander und hintereinander gestapelten Monolithen (4) und vor jeder katalytisch aktiven Zone (5) jeweils eine Mischzone (6) mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher (12) vorgesehen ist
- mit einer oder mehreren Zuführleitungen (7) für den zu dehydrierenden kohlenwasserstoffhaltigen Gasstrom (2),
- mit einer oder mehreren, unabhängig voneinander regelbaren Zuführleitungen (9), wobei jede Zuführleitung (9) eine oder mehrere Verteilerkammern (10) versorgt, für den Sauerstoff enthaltenden Gasstrom (3) in jede der Mischzonen (6) sowie
- mit einer Abführleitung (11) für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagbar ist und
- der zu dehydrierende kohlenwasserstoffhaltige Gasstrom (2) über eine Zuführleitung (7) in den Wärmetauscher (12) eingeleitet, im Wärmetauscher (12) durch das Reaktionsgasgemisch im Gegenstrom durch indirekten Wärmetausch aufgeheizt und weiter an das dem Wärmetauscher (12) entgegengesetzte Ende des Reaktors geleitet, dort umgelenkt, über einen Strömungsgleichrichter (8) in den Innenbereich A eingeleitet, und in den Mischzonen (6) mit dem Sauerstoff enthaltenden Gasstrom (3) vermischt wird, worauf im Innenbereich A des Reaktors (1) die autotherme Gasphasendehydrierung stattfindet,
**dadurch gekennzeichnet, dass**
der Innenbereich A gegenüber dem Außenbereich B des Reaktors mittels eines mikroporösen Hochleistungsdämmstoffes (16) mit einem Wärmeleitfähigkeitswert A bei Temperaturen bis 700 °C von kleiner als 0,05 W/m*K isoliert ist.

2. Reaktor (1) nach Anspruch 1, **dadurch gekennzeichnet, dass** er in Form eines liegenden Zylinders oder Prismas ausgebildet ist.

3. Reaktor (1) nach Anspruch 1 oder 2, **dadurch gekennzeichnet, dass** das Gehäuse G, der Wärmetauscher (12), die Zuführleitung (7) für den zu dehydrierenden kohlenwasserstoffhaltigen Gasstrom (2), sowie die Zuführleitungen (9) für den Sauerstoff enthaltenden Gasstrom (3) gegenüber dem Außenbereich B des Reaktors mittels des mikroporösen Hochleistungsdämmstoffes (16) isoliert sind.

4. Reaktor (1) nach Anspruch 3, **dadurch gekennzeichnet, dass** der mikroporöse Hochleistungsdämmstoff (16) auf der dem Außenbereich B des Reaktors zugewandten Seite des Gehäuses G des Wärmetauschers (12) der Zuführleitung (7) für den zu dehydrierenden kohlenwasserstoffhaltigen Gasstrom (2) sowie der Zuführleitung (9) für den Sauerstoff enthaltenden Gasstrom 3) aufgebracht ist.

5. Reaktor (1) nach einem der Ansprüche 1 bis 4, **dadurch gekennzeichnet, dass** als mikroporöser Hochleistungsdämmstoff (16) ein Werkstoff mit hochdisperser Kieselsäure als Hauptbestandteil und mit einem Trübungsmittel zur Minimierung der Infrarotstrahlung als weiterem Bestandteil in Form von mikroporösen Partikeln mit einer durchschnittlichen Porengröße von etwa 20 nm, eingesetzt wird.

6. Reaktor (1) nach einem der Ansprüche 1 bis 5, **dadurch gekennzeichnet, dass** der mikroporöse Hochleistungsdämmstoff (16) in Form von Platten oder aus Platten hergestellten Formteilen eingesetzt wird.

7. Reaktor (1) nach Anspruch 6, **dadurch gekennzeichnet, dass** die Platten oder die aus Platten hergestellten Formteile mit einer Schicht (17) aus einem Werkstoff ummantelt sind, die die mechanische Stabilität derselben erhöht.

8. Reaktor (1) nach Anspruch 7, **dadurch gekennzeichnet, dass** der Werkstoff, aus dem die Schicht (17) gebildet ist, ein Metall, bevorzugt Edelstahl oder Aluminium ist.

9. Reaktor (1) nach einem der Ansprüche 6 bis 8, **dadurch gekennzeichnet, dass** die aus Platten hergestellten Formteile aus dem mikroporösen Hochleistungsdämmstoff (16) miteinander verzahnbar ausgebildet sind, dergestalt, dass sie unter mechanischer und thermischer Belastung stets eine durchgehende Isolation gewährleisten.

10. Verfahren zur Durchführung einer autothermen Gasphasendehydrierung unter Verwendung eines Reaktors nach einem der Ansprüche 1 bis 9.

11. Verfahren nach Anspruch 10, **dadurch gekennzeichnet, dass** die autotherme Gasphasendehydrierung eine Dehydrierung von Propan, von Butan, von Isobutan, von Buten oder von Ethylbenzol ist.

## Claims

1. A reactor (1) in the form of a cylinder or prism for carrying out an autothermal gas-phase dehydrogenation of a hydrocarbon-comprising gas stream (2) by means of an oxygen-comprising gas stream (3) to give a reaction gas mixture over a heterogeneous catalyst configured as monolith (4), where
- the interior of the reactor (1) is divided by a cylindrical or prismatic gastight housing G which is arranged in the longitudinal direction of the reactor (1) into
- an inner region A having one or more catalytically active zones (5), in which a packing composed of monoliths (4) stacked on top of one another, next to one another and behind one another and before each catalytically active zone (5) in each case a mixing zone (6) having solid internals are provided, and
- an outer region B arranged coaxially to the inner region A, and
- a heat exchanger (12) is provided at one end of the reactor subsequent to the housing G,
- with one or more feed lines (7) for the hydrocarbon-comprising gas stream to be dehydrogenated (2),
- with one or more feed lines (9) which can be regulated independently of one another, where each feed line (9) supplies one or more distribution chambers (10), for the oxygen-comprising gas stream (3) into each of the mixing zones (6) and
- with a discharge line (11) for the reaction gas mixture of the autothermal gas-phase dehydrogenation, where
- the outer region B can be supplied with a gas which is inert under the reaction conditions of the autothermal gas-phase dehydrogenation and
- the hydrocarbon-comprising gas stream (2) to be dehydrogenated is introduced via a feed line (7) into the heat exchanger (12), is heated in the heat exchanger (12) by means of the reaction gas mixture in countercurrent by indirect heat exchange and conveyed further to the end of the reactor opposite the heat exchanger (12), redirected there, introduced via a flow equalizer (8) into the inner region A and mixed with the oxygen-comprising gas stream (3) in the mixing zones (6), whereupon the autothermal gas-phase dehydrogenation takes place in the inner region A of the reactor (1),
wherein
the inner region A is insulated from the outer region B of the reactor by means of a microporous high-performance insulation material (16) having a thermal conductivity λ at temperatures up to 700°C of less than 0.05 W/m*K.

2. The reactor (1) according to claim 1, which is configured in the form of an essentially horizontal cylinder or prism.

3. The reactor (1) according to claim 1 or 2, wherein the housing G, the heat exchanger (12), the feed line (7) for the hydrocarbon-comprising gas stream (2) to be dehydrogenated and the feed lines (9) for the oxygen-comprising gas stream (3) are insulated from the outer region B of the reactor by means of the microporous high-performance insulation material (16).

4. The reactor (1) according to claim 3, wherein the microporous high-performance insulation material (16) has been applied to the side facing the outer region B of the reactor of the housing G, the heat exchanger (12), the feed line (7) for the hydrocarbon-comprising gas stream (2) to be dehydrogenated and the feed line (9) for the oxygen-comprising gas stream (3).

5. The reactor (1) according to any of claims 1 to 4, wherein a material comprising finely divided silica as main constituent and an opacifier to minimize infrared radiation as further constituent in the form of microporous particles having an average pore size of about 20 nm is used as microporous high-performance insulation material (16).

6. The reactor (1) according to any of claims 1 to 5, wherein the microporous high-performance insulation material (16) is used in the form of boards or shaped parts produced from boards.

7. The reactor (1) according to claim 6, wherein the boards or the shaped parts produced from boards are enveloped in a layer (17) of a material which increases the mechanical stability thereof.

8. The reactor (1) according to claim 7, wherein the material of which the layer (17) is made is a metal, preferably stainless steel or aluminum.

9. The reactor (1) according to any of claims 6 to 8, wherein the shaped parts of the microporous high-performance insulation material (16) produced from boards are configured so as to be able to interlock with one another so that they always ensure continuous insulation under mechanical and thermal stress.

10. A process for carrying out an autothermal gas-phase dehydrogenation using a reactor according to any of claims 1 to 9.

11. The process according to claim 10, wherein the autothermal gas-phase dehydrogenation is a dehydrogenation of propane, of butane, of isobutane, of butene or of ethylbenzene.

## Revendications

1. Réacteur (1) en forme de cylindre ou de prisme pour la réalisation d'une déshydrogénation autothermique en phase gazeuse d'un courant de gaz (2) contenant des hydrocarbures avec un courant de gaz (3) contenant de l'oxygène avec obtention d'un mélange de gaz de réaction, sur un catalyseur hétérogène, qui est réalisé sous forme de monolithe (4), dans lequel
- l'espace intérieur du réacteur (1) est divisé par une enceinte étanche au gaz G, de forme cylindrique ronde ou prismatique, disposée dans la direction longitudinale du réacteur (1), en
- une région intérieure A, avec une ou plusieurs zones catalytiquement actives (5), dans lesquelles il est respectivement prévu une garniture composée de monolithes (4) empilés les uns au-dessus, à côté et derrière les uns des autres et respectivement une zone de mélange (6) avec des obstacles fixes avant chaque zone catalytiquement active (5), et
- une région extérieure B disposée coaxialement à la région intérieure A, et dans lequel
- il est prévu à une extrémité du réacteur à la suite de l'enceinte G un échangeur de chaleur (12)
- avec une ou plusieurs conduites d'arrivée (7) pour le courant de gaz contenant des hydrocarbures à déshydrogéner (2),
- avec une ou plusieurs conduites d'arrivée (9) réglables indépendamment l'une de l'autre, dans lequel chaque conduite d'arrivée (9) alimente une ou plusieurs chambres de répartition (10), pour le courant de gaz (3) contenant de l'oxygène dans chacune des zones de mélange (6), ainsi que
- avec une conduite d'évacuation (11) pour le mélange de gaz de réaction de la déshydrogénation autothermique en phase gazeuse, dans lequel
- la région extérieure B peut être alimentée avec un gaz inerte dans les conditions de réaction de la déshydrogénation autothermique en phase gazeuse, et
- le courant de gaz contenant des hydrocarbures à déshydrogéner (2) est introduit dans l'échangeur de chaleur (12) par une conduite d'arrivée (7), chauffé dans l'échangeur de chaleur (12) par le mélange de gaz de réaction à contre-courant par échange de chaleur indirect et transmis à l'extrémité du réacteur opposée à l'échangeur de chaleur (12), dévié à cet endroit, introduit dans la région intérieure A par un redresseur d'écoulement (8), et mélangé dans les zones de mélange (6) avec le courant de gaz contenant de l'oxygène (3), et la déshydrogénation autothermique en phase gazeuse se produit ensuite dans la région intérieure A du réacteur (1), **caractérisé en ce que** la région intérieure A est isolée par rapport à la région extérieure B du réacteur au moyen d'un matériau isolant microporeux à haut rendement (16) présentant un coefficient de conductibilité thermique λ à des températures jusque 700°C de moins de 0,05 W/m*K.

2. Réacteur (1) selon la revendication 1, **caractérisé en ce qu'**il est réalisé à la forme d'un cylindre ou d'un prisme couché.

3. Réacteur (1) selon la revendication 1 ou 2, **caractérisé en ce que** l'enceinte G, l'échangeur de chaleur (12), la conduite d'arrivée (7) pour le courant de gaz contenant des hydrocarbures à déshydrogéner (2) ainsi que les conduites d'arrivée (9) pour le courant de gaz contenant de l'oxygène (3) sont isolés par rapport à la région extérieure B du réacteur au moyen du matériau isolant microporeux à haut rendement (16).

4. Réacteur (1) selon la revendication 3, **caractérisé en ce que** le matériau isolant microporeux à haut rendement (16) est appliqué sur le côté tourné vers la région extérieure B du réacteur de l'enceinte G, de l'échangeur de chaleur (12), de la conduite d'arrivée (7) pour le courant de gaz contenant des hydrocarbures à déshydrogéner (2) ainsi que de la conduite d'arrivée (9) pour le courant de gaz contenant de l'oxygène (3).

5. Réacteur (1) selon l'une quelconque des revendications 1 à 4, **caractérisé en ce que** l'on utilise comme matériau isolant microporeux à haut rendement (16) un matériau avec de l'acide silicique hautement dispersé comme constituant principal et avec un agent opacifiant pour minimiser le rayonnement infrarouge comme autre constituant sous la forme de particules microporeuses avec une taille de pore moyenne d'environ 20 nm.

6. Réacteur (1) selon l'une quelconque des revendications 1 à 5, **caractérisé en ce que** le matériau isolant microporeux à haut rendement (16) est utilisé sous la forme de plaques ou de pièces moulées produites à partir de plaques.

7. Réacteur (1) selon la revendication 6, **caractérisé en ce que** les plaques ou les pièces moulées produites à partir de plaques sont enveloppées d'une couche (17) d'un matériau qui augmente leur stabilité mécanique.

8. Réacteur (1) selon la revendication 7, **caractérisé en ce que** le matériau dont la couche (17) est formée est un métal, de préférence un acier spécial ou l'aluminium.

9. Réacteur (1) selon l'une quelconque des revendications 6 à 8, **caractérisé en ce que** les pièces moulées produites à partir de plaques constituées du matériau isolant microporeux à haut rendement (16) sont réalisées sous une forme s'imbriquant les unes avec les autres, de telle manière qu'elles garantissent toujours une isolation continue sous des charges mécaniques et thermiques.

10. Procédé pour réaliser une déshydrogénation autothermique en phase gazeuse en utilisant un réacteur selon l'une quelconque des revendications 1 à 9.

11. Procédé selon la revendication 10, **caractérisé en ce que** la déshydrogénation autothermique en phase gazeuse est une déshydrogénation de propane, de butane, d'isobutane, de butène ou d'éthylbenzène.
